# EUROPEAN PATENT SPECIFICATION

(11) **EP 2 804 671 B1**
(45) Date of publication and mention of the grant of the patent: **08.03.2017**
(21) Application number: 13701009.6
(22) Date of filing: 16.01.2013
(51) Int. Cl.: A61Q 5/02, A61K 8/892, A61Q 5/12, A61K 8/97, A61K 8/02

(54) **HAIR TREATMENT COMPOSITION**
HAARBEHANDLUNGSZUSAMMENSETZUNG
COMPOSITION DE TRAITEMENT CAPILLAIRE

(30) Priority: 18.01.2012 EP 12151518
(43) Date of publication of application: 26.11.2014
(73) Proprietor: Unilever PLC, London, Greater London EC4Y 0DY (GB); Unilever N.V., 3013 AL Rotterdam (NL)
(72) Inventor: COOKE, Michael, James, Bebington Wirral Merseyside CH63 3JW (GB); RILEY, Robert, George, Bebington Wirral Merseyside CH63 3JW (GB); SHAW, Neil, Scott, Bebington Wirral Merseyside CH63 3JW (GB)
(74) Representative: James, Helen Sarah
(86) International application number: PCT/EP2013/050757
(87) International publication number: WO 2013/107778

(56) References cited:
- WO-A1-2009/101545
- WO-A2-2008/127766
- US-A1- 2005 164 896
- US-A1- 2009 269 376
- KATIE BIRD: "Fiberstar to launch emulsifier alternative for personal care at in-cosmetics", INTERNET CITATION, 16 March 2010 (2010-03-16), page 1, XP002669696, Retrieved from the Internet: URL:http://www.cosmeticsdesign.com/content /view/print/282125 [retrieved on 2012-02-14]
- INDIRA PRABASARI ET AL: "Pectic polysaccharides from mature orange () fruit albedo cell walls: Sequential extraction and chemical characterization", CARBOHYDRATE POLYMERS, APPLIED SCIENCE PUBLISHERS, LTD. BARKING, GB, vol. 84, no. 1, 3 December 2010 (2010-12-03), pages 484-494, XP028151926, ISSN: 0144-8617, DOI: 10.1016/J.CARBPOL.2010.12.012 [retrieved on 2010-12-10]
- FISHER ET AL: "Functional Properties of Herbacel AQ Plus Fruit Fibres", INTERNET CITATION, 13 May 2000 (2000-05-13), pages 1-2, XP008137836, Retrieved from the Internet: URL:http://www.herbafood.de/fileadmin/user _upload/Veroeffentlichungen/Functional_Pro perties_Herbacel_AQ_Plus_Fruit_Fibres.pdf [retrieved on 2011-06-10]
- ARTURO CÃ RDOBA ET AL: "Rheological Behaviour of an Insoluble Lemon Fibre as Affected by Stirring, Temperature, Time and Storage", FOOD AND BIOPROCESS TECHNOLOGY ; AN INTERNATIONAL JOURNAL, SPRINGER-VERLAG, NEW YORK, vol. 5, no. 3, 1 December 2010 (2010-12-01), pages 1083-1092, XP035025626, ISSN: 1935-5149, DOI: 10.1007/S11947-010-0478-2
- GONZALEZ-MOLINA E ET AL: "Natural bioactive compounds of Citrus limon for food and health", JOURNAL OF PHARMACEUTICAL AND BIOMEDICAL ANALYSIS, NEW YORK, NY, US, vol. 51, no. 2, 20 January 2010 (2010-01-20), pages 327-345, XP026691391, ISSN: 0731-7085, DOI: 10.1016/J.JPBA.2009.07.027 [retrieved on 2009-07-29]

## Description

This invention relates to an aqueous shampoo composition comprising water, surfactant, suspending agent and silicone conditioning agent, the suspending agent provides rheological modification to the composition and also suspends the silicone conditioning agent in the liquid.

### Background

Hair shampoos traditionally comprise cleansing surfactants formulated to provide a microstructure of entangled elongated or rod-like micelles. Such microstructures are isotropic and transparent, providing excellent clarity and allowing easy modification of product appearance. However, the native yield stress of such compositions is very low so there is a need for an additional suspending agent if it is desired to include colloidal benefit agents such as oily emulsion droplets such as silicones or particulates such as coated mica or insoluble anti-dandruff agents. Traditionally, suspension in shampoo formulations has been achieved either by adding associative thickeners (polymers that build structure by cross-linking the surfactant micelles) or space-filling particles.

The former class of suspending agent is typified by the Hydrophically-modified Alkali Swellable Emulsion (HASE) polymers such as Aculyn 28 ex Rohm & Haas or Carbopol Aqua SF1 ex-Lubrizol. These polymers comprise a polyacrylate backbone, whose solubility is controlled by pH, and hydrophobic pendant groups that associate with surfactant micelles to form a network. Such networks are typically optically transparent and hence offer a combination of clarity and suspending power. Unfortunately, however, it has been found that, due to the strength of the network thus formed, it may be difficult to break the product down during use. This can have a negative impact on consumer-relevant sensory properties such as foamability and, especially, reduces the efficiency with which the shampoo formulation can release and deposit colloidal benefit agents such as silicone oil droplets.

The latter class of suspending agent can take the form of solid particulates such as ethylene glycol distearate. These particulates are selected to have a high aspect ratio (i.e. they are non-spherical, typically plate-like) as this minimises the amount of suspending material required to achieve the necessary performance. Nonetheless, solid particulates are relatively weight-inefficient and hence relatively expensive suspending agents in shampoo. A more cost-effective alternative is to use microgel particles, in which the bulk of the volume occupied by the particle comprises water. Typical examples of such microgel suspending agents are the cross-linked polyacrylate microgels such as Carbopol 910, Carbopol 934, Carbopol 941 and Carbopol 980 (all ex Lubrizol), which swell within the composition and thus form a space-filling network that can suspend other colloidal materials such as silicone conditioning agents. On application to the hair the networks formed by such microgels fall apart readily to allow efficient deposition of the silicone conditioning material onto the hair. Unfortunately, however, such polyacrylate microgel networks in shampoo are typically opaque and can provide only a relatively dull product appearance.

WO2009/101545 discloses liquid detergent compositions comprising an external structuring system comrpising a bacterial cellulose network.

Thus there remains the need for alternative suspending agents that can suspend materials such as silicone conditioning materials, do not adversely affect the appearance of the product and do not inhibit the deposition of the suspended material from the product.

### Summary of the invention

A shampoo composition comprising:
i) at least 10 wt% of the total composition of water,
ii) at least 0.5 wt% of the total composition of anionic surfactant,
iii) from 0.02 wt% of the total composition to 5 wt% of perfume
iv) at least 0.01 wt% of the total composition a silicone conditioning agent; and
v) a suspending agent comprising at least 0.15 wt%, of the total composition citrus fibre that has been mechanically pulped and swollen in water, in
which the pulped citrus fibre is derived from lemons or limes, and in which the composition further comprises at least 0.05 wt% of the total composition of a cationic deposition polymer.

The invention also describes a method of treating the hair and a method of depositing a silicone conditioning agent by application of the above mentioned composition.

### Description of the Invention

Compositions in accordance with the invention are formulated as compositions for washing the hair and subsequent rinsing.

Shampoo compositions of the invention comprise one or more cleansing surfactants which are cosmetically acceptable and suitable for topical application to the hair. Further surfactants may be present as emulsifiers.

Suitable cleansing surfactants are selected from anionic, amphoteric and zwitterionic surfactants, and mixtures thereof. The cleansing surfactant may be the same surfactant as the emulsifier, or may be different.

### Anionic Surfactant

Compositions of the invention comprise anionic surfactant. Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae R₂OSO₃M and R₁O(C₂H₄O)ₓSO₃M, wherein R₂ is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R₂ has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 wt% to 25 wt% of the total composition, more preferably from 3 wt% to 18 wt%, most preferably from 6 wt% to 15 wt% of the total composition.

The total amount of anionic cleansing surfactant in compositions of the invention generally ranges from 0.5 wt% to 45 wt%, more preferably from 1.5 wt% to 20 wt%.

### Nonionic surfactant

Compositions of the invention may contain non-ionic surfactant. Most preferably non-ionic surfactants are present in the range 0 to 5wt%.

Nonionic surfactants that can be included in compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula R-(OCH₂CH₂)ₙOH, where R is an alkyl chain of C12 to C15, and n is 5 to 9.

Other suitable nonionic surfactants include mono- or di-alkyl alkanolamides. Examples include coco mono- or di-ethanolamide and coco monoisopropanolamide.

Further nonionic surfactants which can be included in compositions of the invention are the alkyl polyglycosides (APGs). Typically, APG is one which comprises an alkyl group connected (optionally via a bridging group) to a block of one or more glycosyl groups. Preferred APGs are defined by the following formula:

RO - (G)n

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

### Amphoteric/zwitterionic Surfactant

Amphoteric or zwitterionic surfactant can be included in an amount ranging from 0.5 wt% to about 8 wt%, preferably from 1 wt% to 4 wt% of the total composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

### Silicone Conditioning Agents

The compositions of the invention contain emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

The viscosity of the emulsified silicone itself (not the emulsion or the final hair conditioning composition) is typically at least 10,000 cst at 25 °C the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of less than 0.15 micron are generally termed microemulsions.

Silicone particle size may be measured by means of a laser light scattering technique, for example using a 2600D Particle Sizer from Malvern Instruments.

Examples of suitable pre-formed emulsions include Xiameter MEM 1785 and microemulsion DC2-1865 available from Dow Corning. These are emulsions/microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning).

Suitable quaternary silicone polymers are described in EP-A-0 530 974. A preferred quaternary silicone polymer is K3474, ex Goldschmidt.

Also suitable are emulsions of amino functional silicone oils with non ionic and/or cationic surfactant.

Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Combination of amino and non amino functional silicones may also be used.

The total amount of silicone is preferably from 0.01 wt% to 10 %wt of the total composition more preferably from 0.1 wt% to 5 wt%, most preferably 0.5 wt% to 3 wt% is a suitable level.

### Pulped Citrus Fibre Suspending Agent

Compositions of the invention comprise 0.15 wt%, of the total composition, preferably 0.2 wt%, of citrus fibre that has been mechanically pulped and swollen in water, in which the pulped citrus fibre is derived from lemons or limes.

More preferably the composition comprises 0.16 to 0.35 wt% of the total composition of pulped citrus fibre derived from lemons or limes.

Citrus fibre is derived from citrus fruit; for the purpose of the present invention, it comprises the peel of lemons and/or limes. The citrus fruit peel is pulped by subjecting it to high shear and the pulped material is referred to as pulped citrus fibre. Such pulped fibres are capable of absorbing and binding at least 15 times its own weight of water, preferably at least 20 times and even up to 30 times.

The suspending agent is a pulped citrus fibre which has undergone a mechanical treatment comprising a step of high intensity mixing in water and which material has consequently absorbed at least 15 times its own dry weight of water, preferably at least 20 times its own weight, in order to swell it. It may be derived by an environmentally friendly process from a fruit processing waste stream. This makes it more sustainable than conventional suspending agents. Furthermore, it requires no additional chemicals to aid its dispersal and it can be made as a structured premix to allow process flexibility.

Citrus fruits are the source of the fibre because they have a large amount of peel that can provide material with the desired water absorbing capacity. The fruits are lemons and limes lemon because the natural pH of the resulting mechanical pulp is about 3.5, which allows use of potassium sorbate at low levels as an effective preservative for the premix before it is dispersed into the detergent liquid.

In a preferred process, the citrus fibre is mechanically pulped by processing it to make a premix preferably in combination with preservative. This is done by adding dried powdered citrus fibre to at least 15 times its own weight of water and dispersing it under very high shear to further break up the citrus fibres and to begin the process of hydration, or swelling. The mechanically treated citrus fibre is left in contact with the water for sufficient time for it to swell due it being fully hydrated. This can be several hours. We have found it advantageous that pulped citrus fibre is kept separate from surfactant until it is fully swollen. This avoids the possibility for the surfactant to compete with the citrus pulp fibre for the water. Something that becomes more of a problem as the total surfactant concentration increases. This premix pulp swelling process seems to become especially advantageous when surfactant level in the composition is 25 wt% or higher. The very high shear may be provided by a high intensity mixer such as a Silverson mixer, or, less preferably, by means of a High-pressure homogeniser. The homogeniser is less preferred because it can suffer from blockage problems with citrus fibre.

The amount of pulped citrus fibre in the premix is preferably from 1 to 5 wt%. More preferably from 2 to 4 wt%. Depending on the processing equipment used there may be a practical upper limit of from 3.3 to 3.5 wt% as it is advantageous that there is excess water in order to fully hydrate the pulped citrus fibre.

It is preferable if the composition comprises less than 0.2 wt% of the composition of a polyacrylic acid based suspending agent, more preferably less than 0.1 wt%, most preferably less than 0.05 wt%.

### Cationic Deposition Polymer

Cationic polymers are ingredients in a shampoo composition of the invention for enhancing deposition performance.

Suitable cationic polymers may be homopolymers which are cationically substituted or may be formed from two or more types of monomers. The weight average (M_{w}) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹)(R²)(R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

### Non-silicone Oily Conditioning Components

Compositions according to the present invention may also comprise a dispersed, non-volatile, water-insoluble oily conditioning agent.

By "insoluble" is meant that the material is not soluble in water (distilled or equivalent) at a concentration of 0.1% (w/w), at 25°C.

Suitable oily or fatty materials are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as C₂-C₆ alkenyl monomers.

Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used.

Particularly preferred fatty esters are mono-, di- and triglycerides, more specifically the mono-, di-, and tri-esters of glycerol and long chain carboxylic acids such as C₁-C₂₂ carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 wt%.

### Further Optional Ingredients

The compositions of the present invention may also contain adjuncts suitable for hair care. Generally such ingredients are included individually at a level of up to 2, preferably up to 1 wt% of the total composition.

Suitable hair care adjuncts include perfumes, fibre actives, antidandruff agents, amino acids, sugars, preservatives, pH adjusters and ceramides.

Particulates such as coated mica or insoluble anti-dandruff agents (insoluble zinc salts such as zinc pyrithione) are preferred adjucts for use with the invention.

The invention will now be further illustrated by the following, non-limiting Examples. Examples of the invention are illustrated by a number, comparative Examples are illustrated by a letter.

**Formula according to the invention (example 1):**

**Shampoo (wt%)**

| **Trade Name** | **Chemical Name** | **Supplier** | **Wt%** |
|---|---|---|---|
| Texapon N701 (70%) | Sodium dodecyl sulphate | Cognis | 17.14 |
| Tegobetaine CK (30%) | Cocamidopropylbetaine | Goldschmidt | 5.33 |
| Timiron MP-1001 (100%) | Mica | S Black | 0.15 |
| Sodium Chloride (100%) | Sodium Chloride | Ellis & Everard | 0.5 |
| Citrus fibre pre-mix (2%) prepared from Herbacel AQ Plus | Citrus fibre production type N, particle size <250• m. [Pre-mix (2% a.i.)] | Herbafood | 12.5 |
| Glydant | DMDM Hydantoin | Lonza | 0.1 |
| Silicone DC 5-7051 HS emulsion (45%) | Dimethiconol, TEA-Dodecylbenzenesulfonate | Dow Corning | 3.78 |
| Jaguar BFG14 (100%) | Guar Hydroxypropyltrimonium Chloride | Rhodia | 0.2 |
| Perfume (100%) | | | 0.9 |
| Water | | | to 100% |

| | | | |
|---|---|---|---|
| Product pH specification: 5.5 -6.5 | | | |

| **Step** | **Processing Instructions for 50 kg batch of the Citrus pulp stabilised shampoo** | | |
|---|---|---|---|
| 1 | Prepare 2% citrus pulp premix. Citrus Pulp raw material is a powder - - Herbacel AQ Plus Citrus Fibres production type N, particle size <250• m. The premix is currently prepared using two processing routes. | | |
| | | 1) Conventional batch processing | |
| | | • | Water along with the citrus pulp powder is charged into a 50L Universal mixer with the recirculation valve closed. The material is then mixed using the scraper (45rpm) and off set turbine (250rpm) for 10 minutes to allow the fibres to hydrate. The premix is then recycled at 5 batch turn overs an hour for 2 hours via a silverson. Silverson mixer ON at 10,000rpm. |
| | | 2) High pressure homogenisation. | |
| | | • | A 2% slurry of the citrus pulp in water is made with a 40L vessel which is mixed using a scraper and off set turbine. The premix is mixed for 30 minutes to allow the fibres to swell. The contents of the vessel are then pumped into the homogeniser at 120 litres per hour and subjected to a processing pressure of 500 bar. |
| | The resulting product from both processing routes has a apple sauce like consistency and appearance. | | |
| 2 | Add 18.239Kg of water to the 50L universal plant along with 6.25Kg of 2% citrus pulp premix that has been manufactured via one of the processing routes above. | | |
| | The mixture is recycled via the silverson ON at 7000rpm for 30 minutes at 5 batch turn overs an hour. | | |
| 3 | 75g of Timiron is dispersed within 1 Kg of water while mixing at 300rpm with a cross paddle. This premix is added over the top into step 2. | | |
| 4 | Texapon N701 (8.571 Kg) is injected inline with the silverson ON at 5000rpm. The mill is switched OFF after all the Texapon has been added. | | |
| 5 | Jaguar BFG14 (100g) is dispersed into the perfume (450g) and added OTT to the main batch and mixed for 10 mins. | | |
| 6 | Add Silicone DC 5-7051 HS (850g) and mix for 5 mins. | | |
| 7 | Inject Tegobetaine CK (2.665g) slowly to avoid gellation and mix for 15 mins. The scraper and off set turbine may have to be increased at this point due to a build in viscosity. | | |
| 8 | Add Glydant (50g) and mix for 10 mins | | |
| 9 | Measure pH - spec 5.5 - 6.5. The product should be in-spec and will require no additional adjustment. | | |
| 10 | In a bucket dissolve Sodium Chloride (250g) in water (2.5Kg). Slowly inject the salt solution into the batch. Mix for 10 mins. | | |
| 11 | Balance the formulation with the remaining water (2Kg). | | |

**Formula not according to the invention (example A):**

**Shampoo (wt%)**

| **Trade Name** | **Chemical Name** | **Supplier** | **Wt**% |
|---|---|---|---|
| Texapon N701 (70%) | Sodium dodecyl sulphate | Cognis | 17.14 |
| Tegobetaine CK (30%) | Cocamidopropylbetaine | Goldschmidt | 5.33 |
| Timiron MP-1001 (100%) | Mica | S Black | 0.15 |
| Sodium Chloride (100%) | Sodium Chloride | Ellis & Everard | 0.5 |
| Carbopol 980 | Polyacrylic acid | BF Goodrich | 0.6 |
| Glydant | DMDM Hydantoin | Lonza | 0.1 |
| Silicone DC 5-7051 HS emulsion (45%) | Dimethiconol, TEA-Dodecylbenzenesulfonate | Dow Corning | 3.78 |
| Jaguar BFG14 (100%) | Guar Hydroxypropyltrimonium Chloride | Rhodia | 0.2 |
| Perfume (100%) | | | 0.9 |
| Water | | | to 100% |

| | | | |
|---|---|---|---|
| Product pH specification: 5.5 -6.5 | | | |

**Formula not according to the invention (example B):**

**Shampoo (wt%)**

| **Trade Name** | **Chemical Name** | **Supplier** | **Wt**% |
|---|---|---|---|
| Texapon N701 (70%) | Sodium dodecyl sulphate | Cognis | 17.14 |
| Tegobetaine CK (30%) | Cocamidopropylbetaine | Goldschmidt | 5.33 |
| Timiron MP-1001 (100%) | Mica | S Black | 0.15 |
| Sodium Chloride (100%) | Sodium Chloride | Ellis & Everard | 0.5 |
| Carbopol Aqua SF1 | Acrylates copolymer | Lubrizol | 0.6 |
| Glydant | DMDM Hydantoin | Lonza | 0.1 |
| Silicone DC 5-7051 HS emulsion (45%) | Dimethiconol, TEA-Dodecylbenzenesulfonate | Dow Corning | 3.78 |
| Jaguar BFG14 (100%) | Guar Hydroxypropyltrimonium Chloride | Rhodia | 0.2 |
| Perfume (100%) | | | 0.9 |
| Water | | | to 100% |

| | | | |
|---|---|---|---|
| Product pH specification: 5.5 -6.5 | | | |

When compared in terms of product appearance, it is found that Example 1 (according to the invention) and Example B (not according to the invention) are both clearly superior to Example A (not according to the invention).

When compared in terms of silicone deposition onto hair, it is found that Example 1 (according to the invention) and Example A (not according to the invention) are both clearly superior to Example B (not according to the invention).

Hence it can be seen that Example 1 (according to the invention) offers the best combination of product appearance characteristics and silicone deposition onto hair.

## Claims

1. A shampoo composition comprising:
i) at least 10 wt% of the total composition of water,
ii) at least 0.5 wt% of the total composition of anionic surfactant,
iii) from 0.02 wt% of the total composition to 5 wt% of perfume
iv) at least 0.01 wt% of the total composition of a emulsified silicone conditioning agent; and
v) a suspending agent comprising at least 0.15 wt%, of the total composition citrus fibre that has been mechanically pulped and swollen in water;
in which the pulped citrus fibre is derived from lemons or limes, and in which the composition further comprises at least 0.05 wt% of the total composition of a cationic deposition polymer.

2. A shampoo composition according to claim 1 in which the level of citrus fibre is preferably at least 0.2wt% of the total composition.

3. A composition according to claim 1 or claim 2 comprising 0.16 to 0.35 wt% pulped citrus fibre.

4. A composition according to any preceding claim in which the level of anionic surfactant is from 1.5 wt% to 20 wt% of the total composition.

5. A composition according to any preceding claim in which the silicone conditioning agent has an average silicone droplet size of less than 10 micron.

6. A method of treating the hair comprising the step of applying to the hair a composition comprising
i) at least 10 wt% of the total composition of water,
ii) at least 0.5 wt% of the total composition of anionic surfactant,
iii) from 0.02 wt% of the total composition to 5 wt% of perfume
iv) at least 0.01 wt% of the total composition of a emulsified silicone conditioning agent; and
v) a suspending agent comprising at least 0.15 wt%, of the total composition citrus fibre that has been mechanically pulped and swollen in water;
in which the pulped citrus fibre is derived from lemons or limes, and in which the composition further comprises at least 0.05 wt% of the total composition of a cationic deposition polymer.

7. A method of depositing an emulsified silicone conditioning agent from a shampoo composition by application of a formulation comprising
i) at least 10 wt% of the total composition of water,
ii) at least 0.5 wt% of the total composition of anionic surfactant,
iii) from 0.02 wt% of the total composition to 5 wt% of perfume
iv) at least 0.01 wt% of the total composition of a emulsified silicone conditioning agent; and
v) a suspending agent comprising at least 0.15 wt%, of the total composition citrus fibre that has been mechanically pulped and swollen in water;
in which the pulped citrus fibre is derived from lemons or limes, and in which the composition further comprises at least 0.05 wt% of the total composition of a cationic deposition polymer.

## Patentansprüche

1. Shampoozusammensetzung, die umfasst:
i) mindestens 10 Gew.-% der gesamten Zusammensetzung an Wasser,
ii) mindestens 0,5 Gew.-% der gesamten Zusammensetzung an anionischem Tensid,
iii) 0,02 bis 5 Gew.-% der gesamten Zusammensetzung an Duftstoff,
iv) mindestens 0,01 Gew.-% der gesamten Zusammensetzung an einem emulgierten Silikonkonditionierungsmittel und
v) ein Suspendierungsmittel, das mindestens 0,15 Gew.-% der gesamten Zusammensetzung an Citrusfaser umfasst, die mechanisch zermahlen und in Wasser gequollen wurde,
in welchem die zermahlene Citrusfaser von Zitronen oder Limonen stammt, und in welcher die Zusammensetzung des Weiteren mindestens 0,05 Gew.-% der gesamten Zusammensetzung an einem kationischen Abscheidungspolymer umfasst.

2. Shampoozusammensetzung nach Anspruch 1, in der der Gehalt an Citrusfaser vorzugsweise mindestens 0,2 Gew.-% der gesamten Zusammensetzung beträgt.

3. Zusammensetzung nach Anspruch 1 oder Anspruch 2, die 0,16 bis 0,35 Gew.-% zermahlene Citrusfaser umfasst.

4. Zusammensetzung nach irgendeinem vorhergehenden Anspruch, in welcher der Gehalt an anionischem Tensid 1,5 bis 20 Gew.-% der gesamten Zusammensetzung beträgt.

5. Zusammensetzung nach irgendeinem vorhergehenden Anspruch, in der das Silikonkonditionierungsmittel eine durchschnittliche Silikontröpfchengröße von weniger als 10 Mikrometer aufweist.

6. Verfahren zur Haarbehandlung, das den Schritt des Aufbringens einer Zusammensetzung auf das Haar umfasst, die umfasst
i) mindestens 10 Gew.-% der gesamten Zusammensetzung an Wasser,
ii) mindestens 0,5 Gew.-% der gesamten Zusammensetzung an anionischem Tensid,
iii) 0,02 bis 5 Gew.-% der gesamten Zusammensetzung an Duftstoff,
iv) mindestens 0,01 Gew.-% der gesamten Zusammensetzung an einem emulgierten Silikonkonditionierungsmittel und
v) ein Suspendierungsmittel, das mindestens 0,15 Gew.-% der gesamten Zusammensetzung an Citrusfaser umfasst, die mechanisch zermahlen und in Wasser gequollen wurde,
in welchem die zermahlene Citrusfaser von Zitronen oder Limonen stammt, und in welcher die Zusammensetzung des Weiteren mindestens 0,05 Gew.-% der gesamten Zusammensetzung an einem kationischen Abscheidungspolymer umfasst.

7. Verfahren zum Abscheiden eines emulgierten Silikonkonditionierungsmittels aus einer Shampoozusammensetzung durch Aufbringen einer Formulierung, die umfasst
i) mindestens 10 Gew.-% der gesamten Zusammensetzung an Wasser,
ii) mindestens 0,5 Gew.-% der gesamten Zusammensetzung an anionischem Tensid,
iii) 0,02 bis 5 Gew.-% der gesamten Zusammensetzung an Duftstoff,
iv) mindestens 0,01 Gew.-% der gesamten Zusammensetzung an einem emulgierten Silikonkonditionierungsmittel und
v) ein Suspendierungsmittel, das mindestens 0,15 Gew.-% der gesamten Zusammensetzung an Citrusfaser umfasst, die mechanisch zermahlen und in Wasser gequollen wurde,
in welchem die zermahlene Citrusfaser von Zitronen oder Limonen stammt, und in welcher die Zusammensetzung des Weiteren mindestens 0,05 Gew.-% der gesamten Zusammensetzung an einem kationischen Abscheidungspolymer umfasst.

## Revendications

1. Composition de shampooing comprenant :
i) au moins 10 % en masse de la composition totale d'eau,
ii) au moins 0,5 % en masse de la composition totale de tensioactif anionique,
iii) de 0,02 % en masse de la composition totale à 5 % en masse de parfum,
iv) au moins 0,01 % en masse de la composition totale d'un agent de conditionnement de silicone émulsionné ; et
v) un agent de suspension comprenant au moins 0,15 % en masse, de la composition totale de fibre d'agrume qui a été mécaniquement réduite en pulpe et gonflée dans de l'eau ;
dans laquelle la fibre d'agrume réduite en pulpe est dérivée de citrons ou de citrons verts, et dans laquelle la composition comprend de plus au moins 0,05 % en masse de la composition totale d'un polymère de dépôt cationique.

2. Composition de shampooing selon la revendication 1, dans laquelle la teneur en fibre d'agrume est de préférence d'au moins 0,2 % en masse de la composition totale.

3. Composition selon la revendication 1 ou la revendication 2 comprenant de 0,16 à 0,35 % en masse de fibre d'agrume réduite en pulpe.

4. Composition selon l'une quelconque des revendications précédentes, dans laquelle la teneur en tensioactif anionique est de 1,5 % en masse à 20 % en masse de la composition totale.

5. Composition selon l'une quelconque des revendications précédentes, dans laquelle l'agent de conditionnement de silicone présente une taille moyenne de gouttelette de silicone inférieure à 10 microns.

6. Procédé de traitement des cheveux comprenant l'étape d'application aux cheveux d'une composition comprenant
i) au moins 10 % en masse de la composition totale d'eau,
ii) au moins 0,5 % en masse de la composition totale de tensioactif anionique,
iii) de 0,02 % en masse de la composition totale à 5 % en masse de parfum
iv) au moins 0,01 % en masse de la composition totale d'un agent de conditionnement de silicone émulsionné, et
v) un agent de suspension comprenant au moins 0,15 % en masse, de la composition totale de fibre d'agrume qui a été mécaniquement réduite en pulpe et gonflée dans de l'eau ;
dans lequel la fibre d'agrume réduite en pulpe est dérivée de citrons ou de citrons verts, et dans laquelle la composition comprend de plus au moins 0,05 % en masse de la composition totale d'un polymère de dépôt cationique.

7. Procédé de dépôt d'un agent de conditionnement de silicone émulsionné à partir d'une composition de shampoing par application d'une formule comprenant
i) au moins 10 % en masse de la composition totale d'eau,
ii) au moins 0,5 % en masse de la composition totale de tensioactif anionique et,
iii) de 0,02 % en masse de la composition totale à 5 % en masse de parfum
iv) au moins 0,01 % en masse de la composition totale d'un agent de conditionnement de silicone émulsionné ; et
v) un agent de suspension comprenant au moins 0,15 % en masse, de la composition totale de fibre d'agrume qui a été mécaniquement réduite en pulpe et gonflée dans de l'eau ;
dans lequel la fibre d'agrume réduite en pulpe est dérivée de citrons ou de citrons verts, et dans laquelle la composition comprend de plus au moins 0,05 % en masse de la composition totale d'un polymère de dépôt cationique.
